# NEUE EUROPÄISCHE PATENTSCHRIFT

(11) **EP 0 298 293 B2**
(45) Veröffentlichungstag und Bekanntmachung des Hinweises auf die Entscheidung über den Einspruch: **24.11.2004**
(45) Hinweis auf die Patenterteilung: 16.03.1994
(21) Anmeldenummer: 88109749.7
(22) Anmeldetag: 18.06.1988
(51) Int. Cl.: A61K 35/60, A61K 31/20, A61K 9/00

(54) **Verfahren zur Herstellung von Fettemulsionen und ihre Verwendung**
Process for the manufacture of fat emulsions and their use
Procédé pour la préparation d'emulsions de graisse et leurs application

(30) Priorität: 08.07.1987 DE 3722540
(43) Veröffentlichungstag der Anmeldung: 11.01.1989
(73) Patentinhaber: Fresenius AG, 61440 Oberursel (DE)
(72) Erfinder: Sommermeyer, Klaus, Dr., D-6365 Rosbach v.d.H. (DE); Weidler, Burghard Priv.-Doz., Dr.-med., D-6365 Rosbach v.d.H. (DE); Sagredos, Angelos N., Prof. Dr., D-2000 Hamburg 20 (DE); Remse, Konrad, D-2000 Hamburg 52 (DE)
(74) Vertreter: Luderschmidt, Schüler & Partner GbR

(56) Entgegenhaltungen:
- WO-A-87/02247
- DE-A- 3 213 744
- DE-A- 3 409 793
- DE-A- 3 409 793
- DE-C3- 2 440 111
- US-A- 4 101 673
- CHEMICAL ABSTRACTS, Band 99, Nr. 10, September 1983, Seite 95, Zusammenfassung Nr. 72560z, Columbus, Ohio, US;
- CHEMICAL ABSTRACTS, Band 106, Nr. 8, 23. Februar 1987, Seite 103, Zusammenfassung Nr. 52095m, Columbus, Ohio, US;
- Physiol, Rev. Vol. 40, 150 (1960)
- Journal of Parenteral and Enteral Nutrition, 230 (1981)
- Progress in the chemistry of fats and other lipids, 57 (1955)

## Beschreibung

Die Erfindung betrifft ein Verfahren zur Herstellung einer hochwertigen Fettemulsion, sowie ihre Verwendung für parenterale und diätetische Zwecke.

Fettemulsionen für parenterale Zwecke dienen der Versorgung von Patienten mit lebensnotwendigen bzw. ergänzenden Nährstoffen, die dem Patienten unter Umgehung des normalen Verdauungsweges gewöhnlich durch intravenöse Infusionen zugeführt werden.

Fettemulsionen für Infusionszwecke sind bereits bekannt. So beschreibt die europäische Patentanmeldung Veröffentlichungs-Nr. 145 873 Emulsionen mit einem Fettgehalt von 10 bis 50 %, in denen die wesentliche Fettkomponente ein α-Linolensäureester, insbesondere das entsprechende Triglycerid, ist.

In der DE-OS 34 09 793 wird eine flüssige Emulsion beschrieben, die eine Fettsäure mit 20 bis 22 Kohlenstoffatomen oder deren Ester oder eine Mischung aus zwei oder mehreren solchen Fettsäuren oder Estern, ferner ein Pflanzenöl, einen Emulgator und Wasser enthält. Die Fettsäuren bzw. deren Ester können in Form eines gereinigten Fischöls eingesetzt werden. Fischöle enthalten bekanntlich mehrfach-ungesättigte Fettsäuren, z.B. Eicosapentaensäure und Docosahexaensäure, in Form von Triglyceriden. In der zitierten Offenlegungsschrift wird zwar darauf hingewiesen, daß die für die Emulsion verwendeten Fischöle vorher gereinigt werden sollen, um nachteilige Reaktionen im lebenden Körper zu vermeiden. Aus dem Beispiel 3 dieser Offenlegungsschrift, das als einziges die Herstellung einer Emulsion unter Verwendung eines Fischöls beschreibt, geht jedoch hervor, daß das dort als gereinigt bezeichnete Sardinenöl gemäß Tabelle 1 auf Seite 9 dieser Offenlegungsschrift einen Gehalt von über 46 % an freien Fettsäuren und von über 10 % an Unverseifbarem (Kohlenwasserstoffe und Sterine) aufweist. Ein solches "gereinigtes" Fischöl enthält demnach trotz durchgeführten Reinigungsverfahrens noch einen hohen Prozentsatz an Bestandteilen, die keine lebensnotwendigen oder ergänzenden Nährstoffe sind und die bei parenteraler Verabreichung unerwünschte Nebenreaktionen verursachen können und daher den Patienten unnötig belasten, wenn sie ihm zugeführt werden.

Im Unterschied zu den Fett- und Ölstoffen, die zur Herstellung von Lebensmitteln, insbesondere von Speisefettprodukten, eingesetzt werden, müssen an die Glyceridöle, die zur Herstellung von Fettemulsionen für Infusionszwecke in der Humanmedizin verwendet werden sollen, aus Gründen der Sicherheit zur Vermeidung gesundheitlicher Schäden beim Patienten besonders hohe Anforderungen in bezug auf die Reinheit der Einzelkomponenten und die gleichbleibende Qualität der Gesamtzusammensetzung gestellt werden. Der DE-OS 34 09 793 sind jedoch keine Hinweise oder Anregungen zu entnehmen, wie hochgereinigt solche Fischöle sein müssen, damit sie im Hinblick auf die Gesundheit des Patienten unbedenklich in Form von Fettemulsionen bei Infusionen angewendet werden können. Die in dem Beispiel 3 dieser Offenlegungsschrift angegebene Zusammensetzung eines gereinigten Sardinenöls entspricht jedenfalls nicht den hohen Qualitätsanforderungen, die an Fettemulsionen für Infusionen gestellt werden müssen.

Neuere analytische Untersuchungen an in herkömmlicher Weise vollraffinierten Glyceridölen haben ergeben, daß diese raffinierten Öle noch merkliche Mengen an Begleitstoffen, insbesondere an dimeren, oligomeren und oxidierten Triglyceriden sowie an Cholesterin, enthalten. Diese unerwünschten Begleitstoffe treten insbesondere bei der Raffination solcher Glyceridöle auf, die, wie z.B. die Fischöle, größere Anteile an mehrfach-ungesättigten Fettsäuren im Triglyceridverband enthalten und dadurch besonders empfindlich gegenüber Oxidations- und Additionsreaktionen sind. Die aufgefundenen Mengen an Begleitstoffen verursachen in der Regel bei der Verwendung dieser Öle in Lebensmitteln keine merkliche Verschlechterung von Geschmack, Geruch und Lagereigenschaften in den Endprodukten. Für höhere Qualitätsansprüche, wie sie für die Verwendung solcher Glyceridöle für diätetische und parenterale Zwecke, insbesondere für Infusionen, gestellt werden müssen, ist es jedoch wünschenswert, Glyceridöle zu verwenden, die möglichst frei von solchen unerwünschten Begleitstoffen sind.

Der vorliegenden Erfindung liegt daher die Aufgabe zugrunde, ein Verfahren zur Herstellung verbesserter Fettemulsionen unter Verwendung von Glyceridölen mit einem Anteil an Eicosapentaensäure und/oder Docosahexaensäure im Triglyceridverband zu schaffen, die mit großer Zuverlässigkeit einen gleichbleibend hohen Qualitätsstandard besitzen und im wesentlichen frei von den oben erwähnten unerwünschten Begleitstoffen sind, so daß diese Fettemulsionen unbedenklich für diätetische und parenterale Zwecke angewendet werden können.

Diese Aufgabe wird erfindungsgemäß gelöst durch das im Anspruch 1 beanspruchte Verfahren.

Bekanntlich enthalten Fischöle Eicosapentaensäure und Docosahexaensäure im Triglyceridverband, die als sogenannte hochungesättigte ω-3-Fettsäuren lebensnotwendige Bausteine sind, die dem Körper zugeführt werden müssen und die beispielsweise als Vorstufe der Prostaglandine und als Strukturelemente der Membranlipide eine wichtige biologische Bedeutung haben. Ferner schreibt man diesen Säuren eine antithrombotische und lipidsenkende Wirkung zu. Da ihre Isolierung aus Naturprodukten und ihre chemische Synthese sehr kostspielig sind, bieten sich die Fischöle als relativ billige Lieferanten dieser essentiellen Fettsäuren an. Ihre Verwendung in Fettemulsionen, die zu parenteralen Zwecken verwendbar sind, setzt aber voraus, daß die Fischöle in einer hochgereinigten Qualität vorliegen, so daß bei einer parenteralen Anwendung gesundheitliche Schäden oder Unverträglichkeiten für die Patienten mit großer Sicherheit vermieden werden können. Aus den vorstehend genannten Gründen ist es ferner erwünscht, daß die hochraffinierten Fischöle angereichert sind mit ω-3-Fettsäuretriglyceriden. Alle diese Voraussetzungen werden mit der vorliegenden Erfindung in hohem Maße erfüllt, wodurch es möglich ist, qualitätsmäßig auf einem sehr hohen Stand befindliche Fettemulsionen für parenterale Zwecke bereitzustellen.

Die erfindungsgemäß hergestellte Fettemulsion zeichnet sich durch einen Gehalt an hochraffiniertem Fischöl aus, das über den im Rohzustand vorhandenen Gehalt hinaus an ω-3-Fettsäuren im natürlichen Triglyceridverband angereichert ist. Dieses Fischöl enthält mindestens 95 Gew.-%, vorzugsweise mindestens 98 Gew.-% monomere Triglyceride, weniger als 2 Gew.-%, bevorzugt weniger als 1 Gew.-% oxidierte Triglyceride, weniger als 0,2 Gew.-%, bevorzugt weniger als 0,1 Gew.-% trimere und oligomere Triglyceride, ferner weniger als 0,8 Gew.-%, bevorzugt weniger als 0,5 Gew.-% dimere Triglyceride und weniger als 1,5 Gew.-%, bevorzugt weniger als 0,8 Gew.-% Unverseifbares, wozu insbesondere Kohlenwasserstoffe und Sterine gerechnet werden. Der Gesamtgehalt des Fischöls an Eicosapentaensäure und Docosahexaensäure im Triglyceridverband liegt im Bereich von 25 bis 50 Gew.-%, vorzugsweise im Bereich von 35 bis 50 Gew.-%, jeweils bestimmt als Flächenprozente im Gaschromatogramm.

Während Fischöle normalerweise einen Cholesteringehalt von etwa 4000 bis 12000 ppm aufweisen, liegt der Gehalt an Cholesterin in den Fischölen der erfindungsgmäßen Fettemulsion bei weniger als 2500 ppm, vorzugsweise bei weniger als 1500 ppm.

Die in dem Fischöl angereicherten ω-3-Fettsäuretriglyceride enthalten als Fettsäurekomponente überwiegend Eicosapentaensäure und Docosahexaensäure. Diese können in verschiedenen Mengenverhältnissen zueinander vorliegen, die durch die Ausmessung der zugehörigen Flächen im Gaschromatogramm (GC) bestimmt werden können. Diese Mengenverhältnisse sind abhängig von der Natur des verwendeten Fischöls und dem Grad der erzielten Anreicherung an ω-3-Fettsäuren. Es hat sich gezeigt, daß Fischöle, bei denen Eicosapentaensäure und Docosahexaensäure im Triglyceridverband in einem Mengenverhältnis vorliegen, das einem GC-Flächenverhältnis von Eicosapentaensäure zu Docosahexaensäure im Bereich von 0,5 bis 2,6 entspricht, Fettemulsionen von ausgezeichneter Qualität liefern, weshalb Mengenverhältnisse in diesem Bereich besonders bevorzugt sind.

Der wesentlichste Bestandteil der erfindungsgemäßen Fettemulsion ist naturgemäß ein hochraffiniertes, mit ω-3-Fettsäuren angereichertes Fischöl, das beispielsweise aus einem Pilchardöl, Menhadenöl, Perufischöl, Sardinenöl, Lachsöl, Heringsöl und/oder Makrelenöl gewonnen werden kann. Zur Herstellung der Emulsionen können Emulgatoren, die physiologisch verträglich sind, beispielsweise gereinigte Lecithine, wie z.B. Sojalecithin, Eilecithin oder Fraktionen daraus, verwendet werden. Außerdem enthält die erfindungsgemäße Fettemulsion zweckmäßigerweise einen Zusatz zur Stabilisierung und Isotonisierung der Fettemulsion, wie z.B. Glycerin, und geringe Mengen an einem Coemulgator, der als Emulgierhilfsmittel wirkt, wie z.B. Alkalisalze der Fettsäuren. Bei den erfindungsgemäßen Fettemulsionen handelt es sich stets um Öl-in-Wasser-(O/W)-Emulsionen, bei denen die äußere, zusammenhängende Phase aus destilliertem, für parenterale Zwecke geeignetem Wasser besteht.

Die genannten Bestandteile können in sehr verschiedenen Mengenverhältnissen in der erfindungsgemäßen Fettemulsion vorliegen. Eine bevorzugte Ausführungsform der erfindungsgemäßen Fettemulsion enthält 5 bis 45 Gew.-% hochraffiniertes, an ω-3-Fettsäuren angereichertes Fischöl, 1 bis 2 Gew.-% Emulgator, 1 bis 3 Gew.-% Emulsionsstabilisator bzw. Isotonisierungszusatz, 0,02 bis 0,05 Gew.-% Coemulgator und als Rest Wasser. Besonders bevorzugt ist eine Fettemulsion mit 8 bis 35 Gew.-% hochraffiniertem, an ω-3-Fettsäuren angereichertem Fischöl, 1 bis 1,5 Gew.-% Emulgator, 1,5 bis 2,5 Gew.-% Emulsionsstabilisator bzw. Isotonisierungszusatz, 0,03 Gew.-% Coemulgator und dem Rest Wasser.

Die Erfindung betrifft ein Verfahren zur Herstellung der Fettemulsion unter Verwendung eines entschleimten, entsäuerten und gebleichten Fischöles mit einem Gehalt an ω-3-Fettsäuren im Triglyceridverband, das dadurch gekennzeichnet ist, daß das Fischöl mit einem mit dem Fischöl mischbaren, flüchtigen Lösungsmittel im Gew./Vol.-Verhältnis Fischöl : Lösungsmittel von 1:1 bis 1:5 vermischt und die Mischung auf eine Temperatur im Bereich von -15 bis -80°C abgekühlt wird, danach vom Ungelösten abfiltriert, das Filtrat schonend vom Lösungsmittel befreit und das so behandelte Fischöl 2 bis 4 Stunden bei 180 bis 220°C gedämpft wird, das gedämpfte Fischöl in einem unpolaren Lösungsmittel aufgenommen und die Lösung über eine mit unpolarem Lösungsmittel eingeschlämmte Kieselgelsäule filtriert wird, anschließend nach schonender Entfernung des unpolaren Lösungsmittels das erhaltene hochraffinierte und an ω-3-Fettsäuren angereicherte Fischöl unter Stickstoffatmosphäre auf 50 bis 60°C erwärmt, durch ein Membranfilter filtriert und dann portionsweise zu einer ebenfalls auf 50 bis 60°C temperierten wäßrigen Mischung, die Emulgator, Emulsionsstabilisator bzw. Isotonisierungszusatz und Coemulgator enthält, unter Rühren zugefügt wird, die gebildete Rohemulsion bei 60 bis 70°C weiter emulgiert und danach unter Stickstoffdruck durch ein Membranfilter filtriert und schließlich einer ein- oder mehrstufigen Homogenisierung bei 70 bis 85°C unterworfen wird, worauf die erhaltene Fettemulsion unter Stickstoff auf eine Temperatur im Bereich von 5 bis 10°C abgekühlt, gegebenenfalls auf einen pH-Wert von 8,5 bis 8,8 eingestellt und in geeigneter Weise unter Sauerstoffausschluß abgefüllt wird.

Die für das erfindungsgemäße Verfahren geeigneten rohen Fischöle werden zunächst in herkömmlicher Weise entschleimt, entsäuert und mit Bleicherden behandelt, wobei die Bedingungen für die einzelnen Verfahrensschritte dem Fachmann bekannt sind und je nach der Zusammensetzung des Fischöls variiert werden können. Daran anschließend wird das Fischöl mit einem mit dem Fischöl mischbaren, flüchtigen Lösungsmittel vermischt, wobei das Gew./Vol.-Verhältnis von Fischöl zu Lösungsmittel im allgemeinen im Bereich von 1:1 bis 1:5, vorzugsweise im Bereich von 1:2 bis 1:3 liegt. Als mit dem Fischöl mischbares, flüchtiges Lösungsmittel hat sich Aceton bewährt, weshalb es in dem Verfahren bevorzugt eingesetzt wird. Die Mischung wird auf eine Temperatur im Bereich von -15 bis -80°C, bevorzugt von -35 bis -60°C, abgekühlt, wobei sich die höherschmelzenden Bestandteile abscheiden. Danach wird vom Ungelösten abfiltriert, das Filtrat schonend vom Lösungsmittel befreit und das erhaltene Fischöl 2 bis 4 Stunden bei 180 bis 220°C, bevorzugt 2 1/2 bis 3 Stunden bei 180 bis 200°C gedämpft. Wegen des hohen Anteils an ungesättigten Fettsäuren im Triglyceridverband des Fischöls muß die thermische Belastung während der Dämpfung möglichst gering gehalten werden, d.h. die üblichen Dämpfungstemperaturen, die bei ca. 200 bis 240°C liegen, und die übliche Dämpfungszeit, die bei 5 bis 7 Stunden liegt, muß nach Möglichkeit deutlich herabgesetzt werden.

Das gedämpfte Fischöl wird in einem unpolaren Lösungsmittel aufgenommen, beispielsweise in Pentan, Hexan, Cyclohexan, Heptan, Petroleumbenzin oder einer Mischung dieser Lösungsmittel. Die Lösung wird über eine ebenfalls mit unpolarem Lösungsmittel eingeschlämmte Kieselgelsäule filtriert, wobei für die Kieselgelsäule ein aktiviertes Kieselgel mit einer Korngröße von etwa 0,05 bis 0,2 mm verwendet wird. Das Gewichtsverhältnis Kieselgel zu Fischöl kann in weiten Grenzen variiert werden, aus wirtschaftlichen Gründen und wegen der leichteren Handhabbarkeit wird die Säulung des Fischöls über Kieselgel bevorzugt mit einem Gewichtsverhältnis Kieselgel zu Fischöl von 1:1 durchgeführt.

Auch die Konzentration des Fischöls in dem unpolaren Lösungsmittel kann in weiten Bereichen variieren, wobei für die Säulung eine Lösung von 5 bis 75 Gew.-% Fischöl in einem unpolaren Lösungsmittel bevorzugt eingesetzt wird.

Aus dem erhaltenen Eluat wird anschließend das unpolare Lösungsmittel schonend entfernt und das erhaltene hochraffinierte und an ω-3-Fettsäuren angereicherte Fischöl unter Stickstoffatmosphäre auf 50 bis 60°C erwärmt. Das erwärmte Fischöl wird durch ein Membranfilter, z.B. ein Nylon-Membranfilter mit einer Porengröße von 0,2 µm, filtriert und dann portionsweise zu einer ebenfalls auf 50 bis 60°C erwärmten wäßrigen Mischung, die Emulgator, Emulsionsstabilisator und Coemulgator enthält, unter Rühren zugefügt. Dabei bildet sich eine Rohemulsion, die bei 60 bis 70 °C weiter emulgiert und danach unter Stickstoffdruck durch ein Membranfilter, z.B. einem solchen mit einem Porendurchmesser von etwa 40 µm, filtriert wird.

Die anschließende Homogenisierung kann ein- oder mehrstufig durchgeführt werden, beispielsweise 4-stufig und bevorzugt in 3 Stufen. Hierbei werden die Temperaturen bei 70 bis 85°C gehalten. In einer besonders bevorzugten 3-stufigen Homogenisierung wird die Temperatur so geführt, daß die Emulsion nach der ersten Homogenisierstufe eine Temperatur von 70°C, nach der zweiten Homogenisierstufe von 80°C und nach der dritten Homogenisierstufe von 80 bis 85 °C aufweist.

Die nach der Homogenisierung erhaltene Fettemulsion wird unter Stickstoff auf eine Temperatur im Bereich von 5 bis 10°C abgekühlt, gegebenenfalls auf einen pH-Wert von 8,5 bis 8,8 eingestellt, z.B. dadurch, daß eine entsprechende Menge 1n Natronlauge zugefügt wird, und danach in geeigneter Weise unter Sauerstoffausschluß abgefüllt, beispielsweise für parenterale Zwecke in Glasbehältnisse, wobei die Fettemulsion vor dem Abfüllen nochmals durch ein Membranfilter, z.B. einem solchen mit einem Porendurchmesser von 2 bis 8 µm, filtriert werden kann.

Die erfindungsgemäßen Fettemulsionen eignen sich hervorragend sowohl für die Herstellung diätetischer Zubereitungen als auch für parenterale Zwecke, insbesondere für Infusionen. Durch das erfindungsgemäße Herstellungsverfahren gelingt es, hochraffinierte und mit ω-3-Fettsäuren, insbesondere Eicosapentaensäure und Docosahexaensäure, im Triglyceridverband angereicherte Fischöle mit nur in engen Grenzen schwankender Zusammensetzung zu Fettemulsionen mit außerordentlich gleichbleibender Qualität zu verarbeiten, so daß die erfindungsgemäßen Fettemulsionen ohne Schwierigkeiten standardisiert werden können. Dies wiederum bedeutet eine praktisch risikolose und sichere Handhabung dieser Fettemulsionen bei parenteralen Anwendungen.

Die Erfindung wird anhand der nachfolgenden Beispiele weiter erläutert.

### Beispiel 1: Herstellung von hochraffiniertem und mit polyungesättigten ω-3-Fettsäuren angereichertem Fischöl

Als Ausgangsprodukt wurde ein rohes Fischöl eingesetzt, das folgende Fettsäurezusammensetzung aufwies:

| Fettsäuren | GC-Flächen-% |
|---|---|
| C_{12:0} | 0,1 |
| C_{14:0} | 7,7 |
| C_{15:0} | 0,7 |
| C_{16:0} | 18,7 |
| C_{16:1} | 6,8 |
| C_{16:2} | 0,7 |
| C_{17:0} | 0,4 |
| C_{18:0} | 3,4 |
| C_{18:1} | 10,6 |
| C_{18:2} | 1,4 |
| C_{18:3} | 0,7 |
| C_{18:4} | 2,8 |
| C_{20:0} | 0,3 |
| C_{20:1} | 1 ,0 |
| C_{20:2} | 0,3 |
| C_{20:3} | 0,1 |
| C_{20:4} | 1,7 |
| C_{20:5} | 13,8 |
| C_{22:0} | 0,1 |
| C_{22:1} | 0,3 |
| C_{22:5} | 2,3 |
| C_{22:6} | 17,8 |
| C_{24:0} | 0,1 |
| C_{24:1} | 0,8 |

Das rohe Fischöl wurde zunächst einer herkömmlichen Entschleimung, alkalischen Entsäuerung und Bleichung unterworfen, wie nachfolgend beschrieben:
a) Entschleimung
100 kg rohes Fischöl wurden unter Stickstoff auf 70°C erhitzt, unter Rühren 1 kg einer 50%igen Citronensäurelösung zugesetzt und 10 Minuten bei 70°C gerührt. Dann kühlte man unter Rühren auf 25 °C ab. Anschließend wurden 1,5 kg Wasser zugesetzt und 60 Minuten bei Raumtemperatur gerührt. Danach wurde die Mischung 20 Minuten bei 4400 U/Min. zentrifugiert und das blanke Öl vom Bodensatz dekantiert.
Ausbeute: 97,5 kg; Säurezahl: 4,6.
b) Alkalische Entsäuerung
97,5 kg entschleimtes Fischöl wurden auf 80°C erhitzt. Unter schnellem Rühren fügte man 2,75 l einer 4n Natronlauge zu. Danach wurde das Rühren eingestellt und 10 l 95°C heißes Wasser aufgebraust. Die Seifenphase wurde abgelassen und die Ölphase zweimal mit je 10 l 0,1n Natronlauge nachgewaschen. Anschließend wurde zweimal mit jeweils 20 l gesättigter Kochsalzlösung, danach einmal mit 20 l Wasser neutral gewaschen. Nach Trocknung des Öls bei 90° C/l hPa wurden 95 kg Öl erhalten.
c) Bleichung
95 kg des entsäuerten Öles wurden mit 1,425 kg (1,5%, bezogen auf Öl) Bleicherde versetzt und 20 Minuten bei 90°C/l hPa gerührt. Nach Filtration über ein Seitzfilter (Seitz-Fimacel 3) wurden 91,7 kg Öl erhalten.
Anschließend an die Bleichung wurde in der nächsten Verfahrensstufe die Anreicherung der ω-3-Fettsäureanteile im Fischöl wie folgt durchgeführt:
d) Anreicherung des gebleichten Fischöls mit ω-3-Fettsäuren im Triglyceridverband
90 kg des entschleimten, entsäuerten und gebleichten Fischöls wurden in 270 Aceton gelöst und die Lösung innerhalb von 4 Stunden unter Rühren auf -15°C abgekühlt und anschließend filtriert. Das Filtrat wurde innerhalb von 4 Stunden weiter auf -35°C abgekühlt und erneut filtriert. Das Filtrat wurde durch Destillation vom Aceton befreit. Man erhielt 13,5 kg Fischöl mit folgenden analytischen Daten:

| Fettsäuren | GC-Flächen-% |
|---|---|
| C_{14:0} | 5,3 |
| C_{15:0} | 0,5 |
| C_{16:0} | 9,4 |
| C_{16:1} | 8,3 |
| C_{16:2} | 1,2 |
| C_{17:0} | 0,1 |
| C_{18:0} | 1,0 |
| C_{18:1} | 10,4 |
| C_{18:2} | 1,5 |
| C_{18:3} | 0,8 |
| C_{18:4} | 3,6 |
| C_{20:1} | 1,0 |
| C_{20:2} | 0,3 |
| C_{20:3} | 0,2 |
| C_{20:4} | 2,7 |
| C_{20:5} | 18,7 |
| C_{22:1} | 0.1 |
| C_{22:5} | 3,0 |
| C_{22:6} | 23,1 |
| C_{24:1} | 0,5 |

1,2 % Unverseifbares
6,2 % oxidierte Triglyceride
0,85 % dimere Triglyceride
0,15 % trimere Triglyceride
9026 mg/kg Cholesterin
Sensorik: Das Produkt roch stark fischig.

e) Dämpfung
13,5 kg des an ω-3-Fettsäuren angereicherten Fischöls wurden 3 Stunden bei 180°C/l hPa mit 5% Wasserdampf-Durchsatz/h gedämpft. Das gedämpfte Fischöl wies die folgenden analytischen Daten auf:
1,2 % Unverseifbares
7,9 % oxidierte Triglyceride
1,95 % dimere Triglyceride
0,45 % trimere Triglyceride
17,8 % Eicosapentaensäure
23,8 % Docosahexaensäure
9217 mg/kg Cholesterin

f) Hochraffination über Kieselgelsäule
13 kg des gedämpften Fischöls wurden in 26 l Hexan gelöst. Die Lösung wurde mit Hilfe einer Pumpe auf eine Kieselgelsäule gegeben, die durch Einschlämmen von 13 kg Kieselgel mit einer Korngröße im Bereich von 0,05 bis 0,2 mm mit 26 l Hexan in eine ca. 50 l fassende Stahlsäule mit einem Durchmesser von 12,5 cm hergestellt worden war. Der Durchsatz der Lösung durch die Kieselgelsäule betrug 5 l/h. Das erhaltene Eluat wurde bei 60°C und 25 hPa weitgehend vom Hexan befreit und das restliche Lösungsmittel im Wasserringvakuum bei 90°C mit Stickstoff gestrippt. Es wurden 10,7 kg eines hochraffinierten, an ω-3-Fettsäuren angereicherten Fischöls mit folgenden analytischen Daten erhalten:

| Fettsäuren | GC-Flächen-% |
|---|---|
| C_{14:0} | 7,2 |
| C_{15:0} | 0,6 |
| C_{16:0} | 13,2 |
| C_{16:1} | 8,5 |
| C_{16:2} | 1,3 |
| C_{17:0} | 1,1 |
| C_{18:0} | 1,6 |
| C_{18:1} | 19,8 |
| C_{18:2} | 1,6 |
| C_{18:3} | 0,8 |
| C_{18:4} | 4,2 |
| C_{20:1} | 0,7 |
| C_{20:4} | 2,3 |
| C_{20:5} | 18,1 |
| C_{22:5} | 2,4 |
| C_{22:6} | 23,3 |

| | |
|---|---|
| Säurezahl | 0,2 |
| Unverseifbares | 0,4 % |
| oxidierte Triglyceride | <0,1 % |
| dimere Triglyceride | 0,4 % |
| trimere Triglyceride | <0,05 % |
| Cholesterin | 1353 mg/kg |
| Sensorik: | kaum wahrnehmbarer Fischgeruch. |

Die Ergebnisse dieses Beispiels zeigen, daß die nach der herkömmlichen Entschleimung, Entsäuerung und Bleichung durchgeführte "Winterisierung" in der Stufe d) eine deutliche Anreicherung an ω-3-Fettsäuren ergibt, daß aber noch eine merkliche Menge an Unverseifbarem, an oxidierten Triglyceriden und an Cholesterin in dem so behandelten Fischöl vorhanden sind und die Sensorik unbefriedigend ist. Die analytischen Daten des gedämpften Fischöls zeigen, daß die Mengen an unerwünschten Begleitstoffen unverändert hoch oder sogar noch erhöht sind, und erst durch die in der letzten Stufe des erfindungsgemäßen Verfahrens erfolgte Behandlung des gedämpften Fischöls mit aktiviertem Kieselgel erhält man ein von den Begleitstoffen praktisch befreites, mit Eicosapentaensäure und Docosahexaensäure hoch angereichertes Fischöl mit einem kaum noch wahrnehmbaren Fischgeruch, das für die erfindungsgemäße Fettemulsion gut geeignet ist.

### Beispiel 2:

Als Ausgangsmaterial wurden 10 kg des gleichen Rohfischöls wie in Beispiel 1 eingesetzt und dieses in der gleichen Weise wie im Beispiel 1 behandelt mit dem Unterschied, daß die zweite Anreicherungsstufe der ω-3-Fettsäuren statt bei -35 °C bei -80 °C durchgeführt wurde. Das nach der Behandlung mit aktiviertem Kieselgel erhaltene Fischöl zeigte folgende Zusammensetzung der Fettsäuren:

| Fettsäuren | GC-Flächen-% |
|---|---|
| C_{12:0} | 0,1 |
| C_{14:0} | 2,8 |
| C_{16:0} | 1,5 |
| C_{16:1} | 8,8 |
| C_{16:2} | 1,2 |
| C_{17:0} | 0,8 |
| C_{18:1} | 10,0 |
| C_{18:2} | 1,6 |
| C_{18:3} | 1,0 |
| C_{18:4} | 4,5 |
| C_{20:1} | 1,0 |
| C_{20:2} | 0,3 |
| C_{20:3} | 0,2 |
| C_{20:4} | 3,0 |
| C_{20:5} | 24,5 |
| C_{22:1} | 0,3 |
| C_{22:5} | 3,3 |
| C_{22:6} | 25,9 |
| C_{24:1} | 0,2 |

Die Behandlung des Fischöls in der zweiten Anreicherungsstufe bei -80 °C statt wie in Beispiel 1 bei -35°C ergibt eine Verschiebung der Anreicherung von polyungesättigten Fettsäuren zugunsten der besonders erwünschten C_{20:5}- und C_{22:6}-Fettsäuren von 18,1 auf 24,5 bzw. 23,3 auf 25,9 GC-Flächenprozente.

### Beispiel 3:

Als Ausgangsmaterial wurde ein rohes Fischöl mit folgender Zusammensetzung der Fettsäuren eingesetzt:

| Fettsäuren | GC-Flächen-% |
|---|---|
| C_{12:0} | 0,2 |
| C_{14:0} | 7,5 |
| C_{16:0} | 17,2 |
| C_{16:1} | 7,9 |
| C_{16:2} | 0,8 |
| C_{17:0} | 0,3 |
| C_{18:0} | 2,3 |
| C_{18:1} | 12,4 |
| C_{18:2} | 1,3 |
| C_{18:3} | 0,9 |
| C_{18:4} | 3,4 |
| C_{20:0} | 0,2 |
| C_{20:1} | 3,4 |
| C_{20:2} | 0,2 |
| C_{20:4} | 0,9 |
| C_{20:5} | 14,7 |
| C_{22:1} | 4,3 |
| C_{22:5} | 2,0 |
| C_{22:6} | 9,6 |
| C_{24:1} | 0,6 |

10 kg dieses Fischöls wurden, wie in Beispiel 1 beschrieben, zunächst in herkömmlicher Weise entschleimt, entsäuert und gebleicht und dann nach dem erfindungsgemäßen Verfahren an ω-3-Fettsäuren angereichert und hochraffiniert mit dem Unterschied, daß in der zweiten Anreicherungsstufe das Gew./Vol.-Verhältnis von Fischöl zu Aceton von 1:3 auf 1:4 geändert und die Mischung statt auf -35 °C nunmehr auf -50°C abgekühlt wurde. Das nach der Säulung über Kieselgel erhaltene Fischöl wies folgende analytische Daten auf:

| Fettsäuren | GC-Flächen-% |
|---|---|
| C_{12:0} | 0,1 |
| C_{14:0} | 3,5 |
| C_{16:0} | 2,4 |
| C_{16:1} | 9,1 |
| C_{16:2} | 1,2 |
| C_{18:0} | 1,1 |
| C_{18:1} | 15,1 |
| C_{18:2} | 2,5 |
| C_{18:3} | 1,3 |
| C_{18:4} | 5,8 |
| C_{20:1} | 2,1 |
| C_{20:2} | 0,2 |
| C_{20:3} | 0,2 |
| C_{20:4} | 2,9 |
| C_{20:5} | 27,9 |
| C_{22:1} | 1,4 |
| C_{22:5} | 2,9 |
| C_{22:6} | 15,1 |
| C_{24:1} | 0,2 |

| | |
|---|---|
| Ausbeute | 7 % |
| Unverseifbares | 0,5 % |
| oxidierte Triglyceride | <0,1 % |
| dimere Triglyceride | 0,4 % |
| trimere Triglyceride | <0,05 % |
| Cholesterin | 1527 mg/kg |

Auch die Ergebnisse dieses Beispiels zeigen, daß das nach dem erfindungsgemäßen Verfahren behandelte Fischöl stark an Eicosapentaensäure und Docosahexaensäure im Triglyceridverband angereichert ist und praktisch frei von unerwünschten Begleitstoffen ist und nur einen relativ geringen Gehalt an Cholesterin aufweist.

### Beispiel 4:

Als Ausgangsmaterial wurde ein rohes Fischöl mit der folgenden Zusammensetzung der Fettsäuren eingesetzt:

| Fettsäuren | GC-Flächen-% |
|---|---|
| C_{12:0} | 0,1 |
| C_{14:0} | 7,9 |
| C_{16:0} | 18,2 |
| C_{16:1} | 10,9 |
| C_{16:2} | 1,4 |
| C_{17:0} | 0,4 |
| C_{18:0} | 2,9 |
| C_{18:1} | 16,4 |
| C_{18:2} | 0,9 |
| C_{18:3} | 0,4 |
| C_{18:4} | 2,4 |
| C_{20:0} | 0,13 |
| C_{20:1} | 0,4 |
| C_{20:2} | 0,1 |
| C_{20:4} | 0,9 |
| C_{20:5} | 17,8 |
| C_{22:1} | 0,1 |
| C_{22:5} | 1,9 |
| C_{22:6} | 7,9 |
| C_{24:1} | 0,3 |

10 kg dieses Fischöls wurden, wie in Beispiel 1 beschrieben, an ω-3-Fettsäuren angereichert und hochraffiniert mit dem Unterschied, daß die Anreicherungsstufe mit einem Gew./Vol.-Verhältnis von Fischöl zu Aceton von 1:4 in der ersten Stufe bei -15°C und in der zweiten Stufe bei -60°C durchgeführt wurde. Das nach der Säulung über eine Kieselgelsäule erhaltene Fischöl zeigte die folgenden analytischen Daten:

| Fettsäuren | GC-Flächen-% |
|---|---|
| C_{14:0} | 3,4 |
| C_{16:0} | 2,3 |
| C_{16:1} | 11,7 |
| C_{16:2} | 2,0 |
| C_{18:0} | 2,6 |
| C_{18:1} | 12,0 |
| C_{18:2} | 1,0 |
| C_{18:3} | 0,5 |
| C_{18:4} | 4,0 |
| C_{20:1} | 0,3 |
| C_{20:2} | 0,1 |
| C_{20:3} | 0,2 |
| C_{20:4} | 2,6 |
| C_{20:5} | 30,5 |
| C_{22:1} | 0,2 |
| C_{22:5} | 2,9 |
| C_{22:6} | 11,9 |

| | |
|---|---|
| Ausbeute | 10 % |
| Unverseifbares | 0,45 % |
| oxidierte Triglyceride | <0,1 % |
| dimere Triglyceride | 0,5 % |
| trimere Triglyceride | <0,05 % |
| Cholesterin | 1412 mg/kg |

Dieses Beispiel zeigt, daß auch bei einem Fischöl, das ursprünglich nur einen mäßigen Gehalt an C_{22:6}-Fettsäure im Triglyceridverband von 7,9 und einen recht hohen Gehalt an C_{20:5}-Fettsäure von 17,8 GC-Flächenprozente aufwies, nach der Behandlung gemäß dem erfindungsgemäßen Verfahren eine ausgezeichnete Anreicherung dieser Fettsäuren auf 11,9 bzw. 30,5 GC-Flächenprozente erzielt werden kann, wobei auch die Mengen an Begleitstoffen bedeutungslos sind und auch der Cholesteringehalt sehr niedrig ist.

In dem folgenden Beispiel wird erläutert, wie unter Verwendung von nach den vorstehenden Beispielen 1 bis 4 hochraffinierten und an ω-3-Fettsäuren angereicherten Fischölen erfindungsgemäße Fettemulsionen erhalten werden.

### Beispiel 5: Herstellung einer erfindungsgemäßen Fettemulsion

Während der gesamten Herstellung der Fettemulsion wurde dafür gesorgt, daß die Komponenten und Mischungen ständig unter Stickstoffatmosphäre gehalten wurden.

Zunächst wurden 12 g Eilecithin in ca. 2 Minuten unter ständigem Rühren in 75 ml destilliertem Wasser für Injektionszwecke, das auf 55 bis 60°C temperiert war, eingegeben und danach die Mischung noch 15 Minuten weitergerührt. Parallel hierzu wurden in 25 ml destilliertem Wasser für Injektionszwecke, das ebenfalls auf 55 bis 60 °C temperiert war, 25 g Glycerin (100-%ig) und 0,3 g Natriumoleat zugegeben und langsam unter Rühren gelöst. Die erhaltene Lösung wurde weiterhin bei 55 bis 60 °C gehalten und mit Stickstoffdruck innerhalb von 10 Minuten durch ein 0,2 µm-Membranfilter in die vorbereitete Wasser/Lecithin-Mischung gegeben.

100 g eines nach den Beispielen 1 bis 4 erhaltenen hochraffinierten und mit ω-3-Fettsäuren angereicherten Fischöls wurden auf 55 bis 60 °C erwärmt und durch ein Nylon-Membranfilter mit einer Porengröße von 0,2 µm innerhalb von 20 bis 25 Minuten direkt in die vorbereitete wäßrige Mischung aus Lecithin, Glycerin und Natriumoleat unter ständigem Rühren, beispielsweise unter Einsatz eines mechanischen Hochfrequenzgerätes (Ultra-Turrax) zum Emulgieren zusammen mit einem Rührwerk, gegeben. Nach beendeter Zugabe des Fischöls wurde die gebildete Rohemulsion 25 Minuten weiter emulgiert. Während der Herstellung der Rohemulsion wurde diese auf einer Temperatur im Bereich von 60 bis 65 °C gehalten und ständig mit Stickstoff überlagert.

Nach Abschalten des mechanischen Hochfrequenzgerätes wurde die Rohemulsion unter leichtem Rühren durch ein Membranfilter mit einer durchschnittlichen Porengröße von 40 µm unter einem Stickstoffdruck von ca. 0,5 bar in einen für die Herstellung von Fettemulsionen geeigneten zweistufigen Homogenisator (1. Stufe 400 bar, 2. Stufe 100 bar) gegeben und homogenisiert. Die Temperatur betrug nach dem ersten Homogenisierschritt etwa 70 °C, nach dem zweiten Homogenisierschritt etwa 80 °C. Die Emulsion wurde anschließend auf 70 °C abgekühlt und in einen Lagertank gegeben, der mit Stickstoff überschichtet war. Hier ließ man die Emulsion unter gelegentlichem langsamen Umrühren ruhen.

Danach wurde die Emulsion noch zwei weiteren Homogenisierschritten unterworfen, wobei die Temperatur nach dem dritten Homogenisierschritt bei etwa 75 °C und nach dem vierten Homogenisierschritt bei 80 bis 85 °C lag. Danach wurde die Emulsion so stark wie möglich abgekühlt und in eine Vorlage gegeben, die 757 ml auf 12°C gekühltes destilliertes Wasser enthielt. Auch dieser Vorgang wurde unter Stickstoffatmosphäre durchgeführt. Unter gelegentlichem langsamen Rühren wurde die Emulsion weiter auf 8 bis 9°C gekühlt. Nach Erreichen dieser Temperatur wurde der Rührer abgeschaltet.

Der pH-Wert der Emulsion wurde geprüft. Abweichungen vom vorgegebenen Sollwert (pH-Wert von 8,7 bis 8,8) wurden durch Zugabe einer entsprechenden Menge an 1 n Natronlauge korrigiert.

Die erhaltene Fettemulsion wurde in einem Kühltank unter Stickstoffatmosphäre aufbewahrt und vor dem Abfüllen durch ein Membranfilter mit einer Porengröße von 2 bis 8 um filtriert. Der Abfülldruck betrug maximal 0,5 bar. Die Abfüllung erfolgte in Glasbehältnisse, die anschließend verschlossen wurden.

Die Fettemulsionen müssen vor Licht und Sauerstoffeinwirkung geschützt werden. Die Abfüllung ist daher unter Stickstoff vorzunehmen, damit der Sauerstoffgehalt in den Behältnissen möglichst gering ist.

Eine toxikologische Prüfung von 10%igen erfindungsgemäßen Fettemulsionen, die in einer einmaligen Dosis von 38,3 ml/kg Körpergewicht und 46,4 ml/kg Körpergewicht intravenös in je 6 männliche und weibliche Sprague-Dawley Ratten injiziert wurden, ergab innerhalb eines Beobachtungszeitraumes von 14 Tagen nach der Applikation keinerlei Hinweise auf eine toxische Wirkung. Die Tiere zeigten einen völlig ungestörten Gesamthabitus und in ihrem Verhalten keine Abweichungen von der Norm. Bei der Sektion der getöteten Tiere nach 14 Tagen wurden keine systemischen und lokalen pathologisch-anatomischen Veränderungen beobachtet.

## Patentansprüche

1. Verfahren zur Herstellung einer parenteral verabreichbaren, für diätetische Zwecke geeigneten Fettemulsion, enthaltend destilliertes Wasser, Emulgator, Emulsionsstabilisator bzw. Isotonisierungszusatz und 5 bis 45 Gew.-% eines hochraffinierten, an ω-3-Fettsäuren im natürlichen Triglyceridverband angereicherten Fischöls mit einem Gehalt von 25 bis 50 Gew.-% an Eicosapentaensäure und Docosahexaensäure, ausgehend von einem entschleimten, entsäuerten und gebleichten Fischöl mit einem Gehalt an ω-3-Fettsäuren im natürlichen Triglyceridverband, **dadurch gekennzeichnet, daß**
a) das Fischöl mit einem mit dem Fischöl mischbaren, flüchtigen Lösungsmittel im Gew./Vol.-Verhältnis Fischöl zu Lösungsmittel von 1:1 bis 1:5 vermischt und die Mischung auf eine Temperatur im Bereich von -15 bis -80°C abgekühlt wird;
b) danach die abgekühlte Mischung vom Ungelösten abfiltriert, das Filtrat schonend vom Lösungsmittel befreit und das so behandelte Fischöl 2 bis 4 Stunden bei 180 bis 220°C gedämpft wird;
c) das gedämpfte Fischöl in einem unpolaren Lösungsmittel aufgenommen und die Lösung über eine mit unpolarem Lösungsmittel eingeschlämmte Kieselgelsäule filtriert wird;
d) anschließend nach schonender Entfernung des Lösungsmittels das erhaltene hochraffinierte und an ω-3-Fettsäuren angereicherte Fischöl unter Stickstoffatmosphäre auf 50 bis 60°C erwärmt, durch ein Membranfilter filtriert und dann portionsweise zu einer ebenfalls auf 50 bis 60°C temperierten wäßrigen Mischung, die Emulgator, Emulsionsstabilisator bzw. Isotonisierungszusatz und Coemulgator enthält, unter Rühren zugefügt wird;
e) die gebildete Rohemulsion bei 60 bis 70°C weiter emulgiert und danach unter Stickstoffdruck durch ein Membranfilter filtriert wird;
f) und schließlich die Emulsion einer ein- oder mehrstufigen Homogenisierung bei 70 bis 85°C unterworfen wird, worauf die erhaltene Fettemulsion unter Stickstoff auf eine Temperatur im Bereich von 5 bis 10°C abgekühlt, gegebenenfalls auf einen pH-Wert von 8,5 bis 8,8 eingestellt und in geeigneter Weise unter Sauerstoffausschluß abgefüllt wird.

2. Verfahren nach Anspruch 1, **dadurch gekennzeichnet, daß** gebleichtes Fischöl und Lösungsmittel, vorzugsweise Aceton, im Gew./Vol.-Verhältnis von 1:2 bis 1:3 eingesetzt werden.

3. Verfahren nach Anspruch 1 oder 2, **dadurch gekennzeichnet, daß** die Mischung aus gebleichtem Fischöl und Lösungsmittel auf eine Temperatur im Bereich von -35 bis -60°C abgekühlt wird.

4. Verfahren nach einem der Ansprüche 1 bis 3, **dadurch gekennzeichnet, daß** die Dämpfung des Fischöls 2 ½ bis 3 Stunden bei 180 bis 200°C durchgeführt wird.

5. Verfahren nach einem der Ansprüche 1 bis 4, **dadurch gekennzeichnet, daß** die Filtration des Fischöls über Kieselgel mit einem Gewichtsverhältnis Kieselgel zu Fischöl von 1:1 durchgeführt und dabei eine Lösung von 5 bis 75 Gew.-% Fischöl in einem unpolaren Lösungsmittel eingesetzt wird.

6. Verfahren nach einem der Ansprüche 1 bis 5, **dadurch gekennzeichnet, daß** die Homogenisierung der filtrierten Emulsion in drei Stufen durchgeführt wird.

7. Verfahren nach Anspruch 6, **dadurch gekennzeichnet, daß** die Homogenisierung so durchgeführt wird, daß die Temperatur der Emulsion nach der ersten Homogenisierstufe bei 70°C, nach der zweiten Stufe bei 80°C und nach der dritten Stufe bei 80 bis 85°C liegt.

8. Verwendung einer nach den Ansprüchen 1 bis 7 hergestellten Fettemulsion zur Herstellung eines Arzneimittels zur parenteralen Anwendung in einem Verfahren zur therapeutischen Behandlung des menschlichen Körpers.

9. Verwendung einer nach den Ansprüchen 1 bis 7 hergestellten Fettemulsion zur Herstellung eines Arzneimittels zur diätetischen Anwendung in einem Verfahren zur therapeutischen Behandlung des menschlichen Körpers.

## Claims

1. Process for the production of a parenterally administrable fat emulsion, suitable for dietetic purposes, containing distilled water, emulsifier, emulsion stabiliser and isotonization additive and 5 to 45 wt.% of a highly refined fish oil enriched with _{ω}-3-fatty acids in the natural triglyceride structure with a content of 25 to 50 wt.% of eicosapentaenoic acid and docosahexaenoic acid in the triglyceride structure, starting from a deslimed, deacidified and bleached fish oil containing _{ω}-3-fatty acids in the natural triglyceride structure; **characterized in that**
a) the fish oil is mixed with a volatile solvent miscible with the fish oil in the wt./vol. ratio of fish oil to solvent of 1:1 to 1:5, and the mixture cooled to a temperature in the range of -15 to -80 °C,
b) then the cooled mixture is filtered off from the insoluble matter, the filtrate being gently freed of the solvent and the thus-treated fish oil steamed for 2 to 4 hours at 180 to 220 °C,
c) the steamed fish oil is absorbed in a non-polar solvent and the solution is filtered over a silica gel column elutriated with a non-polar solvent,
d) then, after gentle removal of the solvent, the obtained highly refined fish oil enriched with _{ω}-3-fatty acids is warmed under nitrogen atmosphere to 50 to 60 °C, filtered through a membrane filter and then added portionwise, with stirring, to an aqueous mixture likewise kept at a controlled temperature of 50 to 60 °C, which contains emulsifier, emulsion stabilisers and isotonization additive and coemulsifier,
e) the formed crude emulsion is further emulsified at 60 to 70 ° C and then filtered through a membrane filter under nitrogen pressure,
f) and finally subjected to single- or multi-step homogenisation at 70 to 85 °C, whereupon the obtained fat emulsion is cooled under nitrogen to a temperature in the range of 5 to 10° C, if necessary set to a pH of 8.5 to 8.8 and drawn off in a suitable way with oxygen exclusion.

2. Process according to claim 1, **characterized in that** bleached fish oil and solvent, preferably acetone, are used in the wt./vol. ratio of 1:2 to 1:3.

3. Process according to claim 1 or 2, **characterized in that** the mixture of bleached fish oil and solvent is cooled to a temperature in the range -35 to -60 ° C.

4. Process according to one of claims 1 to 3, **characterized in that** steaming of the fish oil is carried out for 2½ to 3 hours at 180 to 200 ° C.

5. Process according to one of claims 1 to 4, **characterized in that** the filtration of the fish oil is carried out over silica gel with a weight ratio of silica gel to fish oil of 1:1, and a solution of 5 to 75 wt.% fish oil in a non-polar solvent is used.

6. Process according to one of claims 1 to 5, **characterized in that** the homogenisation of the filtered emulsion is carried out in three stages.

7. Process according to claim 6, **characterized in that** the homogenisation is carried out in such a way that the temperature of the emulsion is 70 ° C after the first homogenisation stage, 80 ° C after the second stage and 80 to 85 °C after the third stage.

8. Use of a fat emulsion produced according to claims 1 to 7 for the production of a pharmaceutical composition for parenteral application in a process for the therapeutic treatment of the human body.

9. Use of a fat emulsion produced according to claims 1 to 7 for the production of a pharmaceutical composition for dietetic application in a process for the therapeutic treatment of the human body.

## Revendications

1. Procédé de préparation d'une émulsion de graisse convenant à des fins diététiques, administrable par la voie parentérale, contenant eau distillée, émulsif, stabilisateur d'émulsion ou additif d'isotonisation et contenant de 5 à 45 % en poids d'une huile de poisson, fortement raffinée, enrichie en acides
ω-3-gras en association naturelle triglycéridique présentant une teneur de 25 à 50 % en poids en acide eicosapentaénoique et en acide docosahexaénoique, au départ d'une huile de poisson démucilaginée, désacidifiée et blanchie, contenant des acides ω-3-gras en association naturelle triglycéridique **caractérisé en ce que**
a) on mélange l'huile de poisson avec un solvant volatil, miscible à l'huile de poisson, dans le rapport pondéral/volumique de l'huile de poisson au solvant de 1 :1 à 1 :5 et on refroidit le mélange jusqu'à une température qui se situe dans la plage de -15 à -80 °C,
b) puis on débarrasse le mélange refroidi des substances non dissoutes par filtration, on débarrasse le filtrat de manière ménagée du solvant et on évapore l'huile de poisson ainsi traitée à 180 - 220 °C pendant 2 à 4 heures,
c) on reprend l'huile de poisson évaporée dans un solvant apolaire et on filtre la solution à travers une colonne de gel de silice transformée en boue à l'aide de solvant apolaire,
d) puis, après élimination ménagée du solvant, on chauffe l'huile fortement raffinée et enrichie en acides ω-3-gras à 50 - 60 °C sous atmosphère d'azote, on la filtre à travers un filtre à membrane et on l'ajoute ensuite, par fractions, à un mélange aqueux, également porté à une température de 50 à 60 °C, qui contient émulsif, stabilisateur d'émulsion ou additif d'isotonisation et coémulsif, sous agitation,
e) on émulsionne davantage l'émulsion brute formée à 60 - 70 °C et on la filtre ensuite à travers un filtre à membrane sous pression d'azote et
f) on la soumet finalement à une homogénéisation en une ou plusieurs étapes à 70 - 85 °C, puis on refroidit l'émulsion de graisse obtenue sous atmosphère d'azote jusqu'à une température qui fluctue dans la plage de 5 à 10 °C, on en règle éventuellement la valeur du pH à 8,5 - 8,8 et on la conditionne d'une manière connue sous exclusion d'oxygène.

2. Procédé suivant la revendication 1, **caractérisé en ce que** l'on utilise l'huile de poisson blanchie et le solvant, de préférence l'acétone, dans le rapport pondéral / volumique de 1 :2 à 1 :3.

3. Procédé suivant la revendication 1 ou 2, **caractérisé en ce que** l'on refroidit le mélange d'huile de poisson blanchie et de solvant jusqu'à une température qui se situe dans la plage de -35 à -60 °C.

4. Procédé suivant l'une quelconque des revendications 1 à 3, **caractérisé en ce que** l'on entreprend l'évaporation de l'huile de poisson à 180 - 200 °C pendant une période qui varie de 2,5 à 3 heures.

5. Procédé suivant l'une quelconque des revendications 1 à 4, **caractérisé en ce que** l'on entreprend la filtration de l'huile de poisson sur du gel de silice avec un rapport pondéral du gel de silice à l'huile de poisson de 1:1 et on met ainsi en oeuvre une solution de 5 à 75 % en poids d'huile de poisson dans un solvant apolaire.

6. Procédé suivant l'une quelconque des revendications 1 à 5, **caractérisé en ce que** l'on entreprend l'homogénéisation de l'émulsion filtrée en trois étapes.

7. Procédé suivant la revendication 6, **caractérisé en ce que** l'on entreprend l'homogénéisation de manière à ce que la température de l'émulsion soit de 70 °C après la première étape d'homogénéisation, de 80 °C après la seconde étape et de 80 à 85 °C après la troisième étape.

8. Utilisation d'une émulsion de graisse préparée suivant les revendications 1 à 7, pour la préparation d'un moyen pharmaceutique destiné à l'utilisation parentérale dans un procédé de traitement thérapeutique du corps humain.

9. Utilisation d'une émulsion de graisse préparée suivant les revendications 1 à 7, en vue de l'utilisation diététique dans un procédé de traitement thérapeutique du corps humain.
